# EUROPÄISCHE PATENTSCHRIFT

(11) **EP 0 388 590 B1**
(45) Veröffentlichungstag und Bekanntmachung des Hinweises auf die Patenterteilung: **02.04.1997**
(21) Anmeldenummer: 90101285.6
(22) Anmeldetag: 23.01.1990
(51) Int. Cl.: G01N 31/00

(54) **Verfahren zum Nachweis von zersetzungsfähigen organischen Kohlenstoffverbindungen, die in einer gasförmigen Phase vorliegen**
Method for determining degradable organic matter in a gaseous phase
Procédé pour déterminer des composés organiques dégradables dans une phase gazeuse

(30) Priorität: 21.03.1989 DE 3909227
(43) Veröffentlichungstag der Anmeldung: 26.09.1990
(73) Patentinhaber: HÜLS AKTIENGESELLSCHAFT, 45764 Marl (DE)
(72) Erfinder: Duve, Hans, Dipl.Ing., D-4408 Dülmen (DE)

(56) Entgegenhaltungen:
- DE-A- 2 823 587
- FR-A- 2 408 139
- GB-A- 2 174 364
- US-A- 4 293 522

## Beschreibung

Die Erfindung betrifft ein Verfahren zum kontinuierlichen Nachweis von zersetzungsfähigen organischen Verbindungen, die in einer gasförmigen Phase vorliegen.

Sie verfolgt den Zweck, die in einer Gasphase vorhandenen organischen Verbindungen auch in sehr kleinen Konzentrationen auf einfache Weise nachzuweisen. Dabei soll sowohl der Gehalt einer Gasphase an organisch gebundenem Kohlenstoff als auch der Gehalt an organisch gebundenen Heteroatomen (z. B. Chlor, Fluor) zugänglich sein. Solche Überwachungen sind beispielsweise notwendig für Gase, falls darin Verbindungen vermutet werden, die störende Auswirkungen haben können. Die zu untersuchenden Gasströme können repräsentative Teilmengen eines großen Gasvolumens darstellen (z. B. Raumluft, Sauerstoff, Inertgase, Abgase); ebenso ist es möglich Strippgase zu untersuchen, die durch eine flüssige Phase geführt wurden und dabei aus dieser Phase organische Verbindungen ausgetrieben haben.

In DE-PS 26 03 752 wird ein diskontinuierliches Verfahren zur Bestimmung von organisch gebundenem Kohlenstoff in wäßriger Lösung beschrieben. Eine wäßrige Probe wird in reines Wasser als Trägerflüssigkeit eingegeben, dieses Gemisch in einem Kreislauf durch einen Photo-Reaktor mit ultraviolettem Licht (UV-Reaktor) geleitet und das bei der Zersetzung der organischen Verbindungen entstandene Kohlendioxid (CO₂) mit einem Gasstrom aus der Trägerflüssigkeit ausgetrieben, in einem abgeschlossenen Teilvolumen der Trägerflüssigkeit rückgelöst und hier mit einer Leitfähigkeitsmessung nachgewiesen. Nach Beendigung der Untersuchung einer Probe werden die Flüssigkeitsinhalte der beiden Teilvolumina durch kontinuierliches Durchleiten durch einen Ionenaustauscher zu reinem Wasser regeneriert. Dieses Verfahren ist beschränkt auf die Untersuchung von wäßrigen Einzelproben, wobei nur das aus den organischen Verbindungen entstandene flüchtige Zersetzungsprodukt CO₂ nachgewiesen wird.

In DE-PS 32 23 167 wird ein Verfahren beschrieben zum Untersuchen von Wasser, das zersetzungsfähige Kohlenstoffverbindungen enthält. Durch einen UV-Reaktor wird kontinuierlich ein wäßriger Probestrom hindurchgeleitet, und die nichtflüchtigen Zersetzungsprodukte, die aus den organischen Verbindungen entstanden sind, werden im wäßrigen Probestrom bei dessen Austritt aus dem UV-Reaktor mit einer Leitfähigkeitsmessung in diesem Probestrom nachgewiesen. Bei diesem Meßverfahren werden auch die nichtflüchtigen Zersetzungsprodukte aus organischen Verbindungen, die in einem wäßrigen Probestrom enthalten sind, nachgewiesen, jedoch ist dieses Verfahren nicht geeignet, um wäßrige Einzelproben zu untersuchen.

GB-A-2 174 364 beschreibt eine Vorrichtung und ein Verfahren zur diskontinuierlichen Bestimmung des Gehalts an gelösten organischen Kohlenstoffverbindungen in flüssigen, wäßrigen Einzelproben. Die gelösten organischen Kohlenstoffverbindungen werden durch UV-Bestrahlung zersetzt; über das dabei entstehende Kohlendioxid wird der Gehalt an organischen Kohlenstoffverbindungen in der flüssigen Probe bestimmt.

Damit stellt sich die Aufgabe, ein Verfahren zu finden, mit dem man die in einer Gasphase enthaltenen organischen Verbindungen unter Miterfassung der nicht kohlenstoffhaltigen Zersetzungprodukte auf einfache Weise, mit hoher Empfindlichkeit und vollständig nachweisen kann.

Die Aufgabe wird erfindungsgemäß gelöst durch ein Verfahren, das dadurch gekennzeichnet ist, daß man die zu untersuchende gasförmige Phase in eine begrenzte Wassermenge einbringt, die im Probegut enthaltenen organischen Kohlenstoffverbindungen in einem UV-Reaktor bestrahlt, der wenigstens einen Teil der begrenzten Wassermenge enthält, und die bei der Zersetzung der organischen Kohlenstoffverbindungen gebildeten Zersetzungsprodukte nachweist.

Der UV-Reaktor ist mit einer begrenzten Wassermenge gefüllt. Diese Wassermenge kann in einem externen Kreislauf mit einer Pumpe umgewälzt werden. Das Kreislaufwasser durchläuft also ständig den UV-Reaktor, wobei die im Kreislaufwasser enthaltenen organischen Verbindungen photochemisch zersetzt und ionisiert werden. Der Gehalt des Kreislaufwassers an Ionen kann im Kreislaufwasser, das den UV-Reaktor verläßt, mit einem Ionendetektor nachgewiesen werden. Wird das Kreislaufwasser vor dem Rückführen in den UV-Reaktor über einen Ionenaustauscher geführt, werden die im Kreislaufwasser enthaltenen Ionen kontinuierlich aus diesem entfernt. Durch einen niedrigen und konstanten Meßwert am Ionendetektor hinter dem UV-Reaktor wird angezeigt, daß das Kreislaufwasser frei ist von zersetzungsfähigen organischen Verbindungen und anorganischen Ionen. In dieses so vorbereitete volumenmäßig begrenzte Kreislaufwasser kann eine zu untersuchende gasförmige Phase eingebracht werden. Die Stelle, an dem die gasförmige Phase in das Kreislaufwasser eingebracht wird, hat einen erheblichen Einfluß auf den sich ergebenden Meßeffekt, der sich bei Anwesenheit von organischen Verbindungen in der gasförmigen Phase einstellt.

Die zu untersuchende gasförmige Phase kann in den unteren Teil des UV-Reaktors in das darin befindliche Kreislaufwasser, welches den UV-Reaktor ständig durchfließt, eingeperlt werden. Im oberen Teil des UV-Reaktors verläßt die gasförmige Phase den Reaktor, wogegen das Kreislaufwasser über einen Ionendetektor geführt wird, ein im Kreislauf ggf. vorhandenes Kreislaufwasservorratsgefäß durchläuft, und über einen Ionenaustauscher mit einer im Kreislauf vorhandenen Pumpe in den unteren Teil des UV-Reaktors rückgeführt wird. Die über die gasförmige Phase in den UV-Reaktor eingebrachten organischen Verbindungen werden unter Einfluß der UV-Bestrahlung in Kohlendioxid und ggf. nicht kohlenstoffhaltige Zersetzungsprodukte umgewandelt. Die nicht kohlenstoffhaltigen Zersetzungsprodukte (z. B. Chlorid-, Fluorid-Ionen) verbleiben vollständig im Kreislaufwasser und können mit einem Ionendetektor ggf. ionensensitiv nachgewiesen werden. Der Gehalt des Kreislaufwassers an CO₂ im Ablauf des UV-Reaktors ist von verschiedenen Faktoren abhängig.

Bevorzugt sollte die in den UV-Reaktor eingeleitete gasförmige Phase frei von CO₂ sein oder vor dem Einleiten mit einem CO₂-Adsorber von CO₂ befreit werden. In diesem Falle wird das bei der Zersetzung der organischen Verbindungen entstandene CO₂ fast vollständig aus dem Kreislaufwasser entfernt. Als CO₂-Adsorber sind Natronkalk und Natronasbest geeignet. Werden als CO₂-Adsorber gekörnter Natronkalk oder Natronkalk-Plätzchen eingesetzt, ist es zweckmäßig, diese vorher mit Wasser anzufeuchten. Durch das einmalige Anfeuchten wird die Adsorption von organischen Kohlenstoffverbindungen aus dem Trägergas verhindert. Diese Eigenschaft der Natronkalk-Plätzchen bleibt überraschenderweise nach dem Abtrocknen im Trägergasstrom erhalten.

Die gasförmige Phase erfüllt damit folgende Funktionen:
- Zuführen der nachzuweisenden organischen Verbindungen in das Kreislaufwasser des UV-Reaktors.
- Abführen des bei der Zersetzung der organischen Verbindungen entstandenen Kohlendioxids aus dem Kreislaufwasser des UV-Reaktors, bevor dieses über den Ionendetektor geführt wird.

In der den UV-Reaktor verlassenden Gasphase kann das bei der Zersetzung der organischen Verbindungen entstandene CO₂ mit einem CO₂-Detektor nachgewiesen werden.

Wird pro Zeiteinheit eine konstante Menge zersetzungsfähiger heteroatomhaltiger organischer Verbindungen über die gasförmige Phase in das im UV-Reaktor befindliche Wasser eingebracht, kann durch eine Verringerung der durch den UV-Reaktor geleiteten Kreislaufwassermenge pro Zeiteinheit der Gehalt an nicht kohlenstoffhaltigen Zersetzungsprodukten im Wasser des UV-Reaktors angereichert werden. Dieser Anreicherungseffekt wird zu einer integralen Größe, wenn während der Zuführung des gasförmigen Probegutes die begrenzte Wassermenge, die sich im UV-Reaktor befindet, nicht kontinuierlich regeneriert wird und sich der Ionendetektor direkt im Wasser des UV-Reaktors befindet.

Im Gegensatz zu der Anreicherung der nicht kohlenstoffhaltigen Zersetzungsprodukte im Wasser des UV-Reaktors wird das bei der Zersetzung der organischen Verbindung entstandene CO₂ kontinuierlich aus dem Wasser des UV-Reaktors ausgetrieben; es kann in der den UV-Reaktor verlassenden Gasphase mit einem CO₂-Detektor als differenzieller Wert nachgewiesen werden.

Wird die zu untersuchende Gasphase durch das Wasser des im Kreislauf befindlichen Vorratsgefäßes geleitet, lösen sich alle in der Gasphase vorhandenen Verbindungen proportional ihrem Verteilungskoeffizienten (flüssige Phase/gasförmige Phase) in diesem Wasser. Für jeden Inhaltsstoff der Gasphase stellt sich im Wasser des Vorratsgefäßes ein dynamisches Gleichgewicht ein. Wird das Kreislaufwasser, das das Vorratsgefäß verläßt, über einen Ionenaustauscher geleitet, werden alle aus der Gasphase gelösten ionenbildenden Bestandteile (z. B. CO₂, NH₃, HCl) aus dem Kreislaufwasser entfernt, bevor dieses durch den UV-Reaktor geleitet wird. Alle bei der Zersetzung der organischen Verbindungen entstandenen Produkte verbleiben im Kreislaufwasser und können mit einem Ionendetektor im Kreislaufwasser hinter dem UV-Reaktor nachgewiesen werden.

Enthält die zu untersuchende Gasphase Verbindungen, die in wäßriger Lösung ohne UV-Bestrahlung Ionen bilden, und werden diese Verbindungen beim Durchleiten der Gasphase durch das Wasser im Vorratsgefäß vollständig aus der Gasphase entfernt, dann kann es vorteilhaft sein, die das Vorratsgefäß verlassende Gasphase durch das Wasser des UV-Reaktors zu leiten. In diesem Falle können wiederum die entstandenen Zersetzungsprodukte in der den UV-Reaktor verlassenden Gasphase und im Kreislaufwasser hinter dem UV-Reaktor nachgewiesen werden.

In allen Fällen kann das den UV-Reaktor verlassende Kreislaufwasser vor dem Ionennachweis mit einem Ionendetektor über einen selektiven Ionenaustauscher geleitet werden, wodurch bestimmte Ionenarten vor deren Nachweis aus dem Kreislaufwasser entfernt werden.

Werden in einer flüssigen Phase organische Verbindungen vermutet, die sich mit einem hindurchgeführten Trägergasstrom aus dieser entfernen lassen, kann anschließend dieser Trägergasstrom mit den darin enthaltenen, aus der flüssigen Phase ausgestrippten organischen Verbindungen als gasförmiges Probegut in die begrenzte Wassermenge eingebracht und untersucht werden. Es kann vorteilhaft sein, den pH-Wert der flüssigen Phase vor dem Durchleiten des Trägergasstromes durch Zugeben von Säure oder Lauge so einzustellen, daß ggf. in der flüssigen Phase vorhandene ausgasbare anorganische Verbindungen (z. B. CO₂, NH₃) in Verbindungen überführt werden, die nicht mehr in den Trägergasstrom übergehen können.

Als Trägergasstrom wird bevorzugt Luft eingesetzt, die frei von kohlenstoffhaltigen Substanzen ist. Vorteilhaft ist jedoch ein von molekularem Sauerstoff freies Trägergas, vorzugsweise Stickstoff, oder ein Edelgas wie z. B. Helium oder Argon.

Das erfindungsgemäße Verfahren kann beispielsweise mittels der in den Figuren dargestellten Vorrichtungen durchgeführt werden.

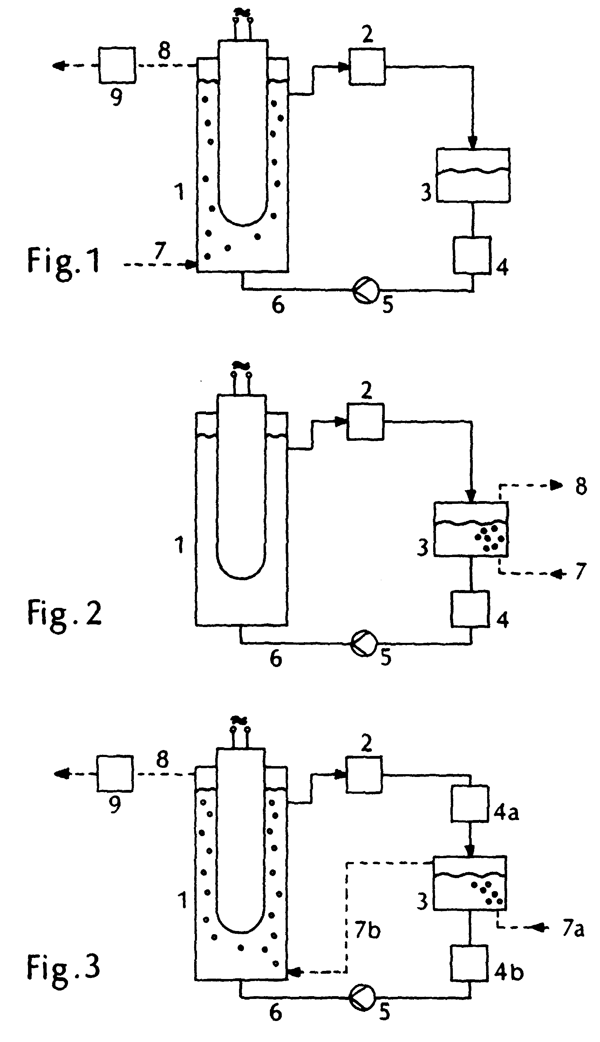

In der Figur 1 besteht der Wasserkreislauf aus UV-Reaktor (1), Ionendetektor (2), Vorratsgefäß (3), Ionenaustauscher (4) und der Kreislaufpumpe (5). Über die Kreislaufleitung (6) wird das Kreislaufwasser ständig umgewälzt. Im unteren Teil des UV-Reaktors wird der zu untersuchende Gasstrom in das im UV-Reaktor befindliche Wasser über die Gas-Zuleitung (7) eingeperlt. Über die Gas-Ableitung (8) verläßt der Gasstrom den UV-Reaktor und kann ggf. durch einen CO₂-Detektor (9) geleitet werden. Die über den Gasstrom in den UV-Reaktor eingebrachten organischen Verbindungen werden in CO₂ und ggf. nicht kohlenstoffhaltige Produkte zersetzt. Das entstehende CO₂ wird mit dem Gasstrom, der durch das Wasser des UV-Reaktors geleitet wird, praktisch vollständig aus diesem Wasser entfernt und kann in dem den UV-Reaktor verlassenden Gasstrom nachgewiesen werden. Die nichtflüchtigen Zersetzungsprodukte werden im Kreislaufwasser, das den UV-Reaktor verläßt, mit einem Ionendetektor nachgewiesen. Der Trennungseffekt kann durch Verlängern der Verweilzeit des Kreislaufwassers im UV-Reaktor verstärkt werden. In diesem Fall kann es vorteilhaft sein, den Ionendetektor direkt in das bestrahlte Wasser im UV-Reaktor einzusetzen. Das den Ionendetektor verlassenden Kreislaufwasser durchläuft das Vorratsgefäß und anschließend einen Ionenaustauscher, womit alle im Kreislaufwasser enthaltenen ionogenen Verbindungen aus diesem entfernt werden. Hinter dem Ionenaustauscher wird das ionenfreie Kreislaufwasser mit einer Pumpe in den unteren Teil des UV-Reaktors gefördert.

In der Figur 2 ist der Wasserkreislauf aufgebaut wie in Figur 1, jedoch wird der zu untersuchende Gasstrom über die Gas-Zuleitung (7) in das im Vorratsgefäß befindliche Kreislaufwasser eingeperlt. Über die Gas-Ableitung (8) verläßt der Gasstrom das Ausgasegefäß. Im Kreislaufwasser des Vorratsgefäßes lösen sich entsprechend der Lösungsgleichgewichte die Bestandteile des zugeführten Gasstromes.

Die aus dem Gasstrom in Lösung gegangenen ionenbildenden Bestandteile (z. B. CO₂) werden über den Ionenaustauscher (4) aus dem Kreislaufwasser entfernt. Vor dem Ionenaustauscher können ggf. die Ionen mit einem Ionendetektor nachgewiesen werden. Alle im Kreislaufwasser gelösten nichtionogenen Bestandteile werden mit dem Kreislaufwasser in den UV-Reaktor (1) gefördert. Die durch die UV-Bestrahlung aus den organischen Verbindungen entstandenen ionogenen Zersetzungsprodukte durchlaufen hinter dem UV-Reaktor den Ionendetektor (2) und werden hier nachgewiesen. Wird vor dem Ionendetektor, der beispielweise eine Leitfähigkeitsmeßzelle oder eine ionenselektive Elektrode sein kann, ein selektiver Ionenaustauscher durchlaufen, so können vor der Messung spezielle Ionenarten aus dem Kreislaufwasser entfernt werden. Über das Vorratsgefäß wird das Kreislaufwasser wieder dem Ionenaustauscher zugeführt und hier von allen ionogenen Inhaltsstoffen befreit. Enthält der zu untersuchende Gasstrom keine ohne UV-Bestrahlung ionenbildenden Bestandteile, kann der Ionenaustauscher (4) auch direkt hinter dem Ionendetektor (2) angeordnet sein.

In Figur 3 wird der zu untersuchende Gasstrom nach dem Einleiten in das Wasser des Vorratsgefäßes über die Leitung (7a) mittels Leitung (7b) in das Wasser des UV-Reaktors eingeperlt. Über Leitung (8) verläßt der Gasstrom den UV-Reaktor und kann durch einen CO₂-Detektor (9) geleitet werden, um das bei der Zersetzung der organischen Verbindungen entstandene CO₂ nachzuweisen. Die nach dem UV-Reaktor im Kreislaufwasser verbleibenden ionogenen Verbindungen werden mit dem Ionendetektor (2) nachgewiesen und anschließend mit dem Ionenaustauscher (4a) aus dem Kreislaufwasser entfernt. Enthält die in das Wasser des Vorratsgefäßes eingeleitete Gasphase u. a. in wäßriger Lösung ionenbildende Bestandteile, deren Löslichkeitsgleichgewicht praktisch vollständig auf der Seite der wäßrigen Lösung liegt (z. B. Chlorwasserstoff), so können diese im Wasser des Vorratsgefäßes aus der Gasphase entfernt werden. Diese nun im Kreislaufwasser gelösten ionogenen Bestandteile werden im Ionenaustauscher (4b) (evtl. nach Detektion) aus dem Kreislaufwasser entfernt, bevor dieses in den UV-Reaktor eingeleitet wird.

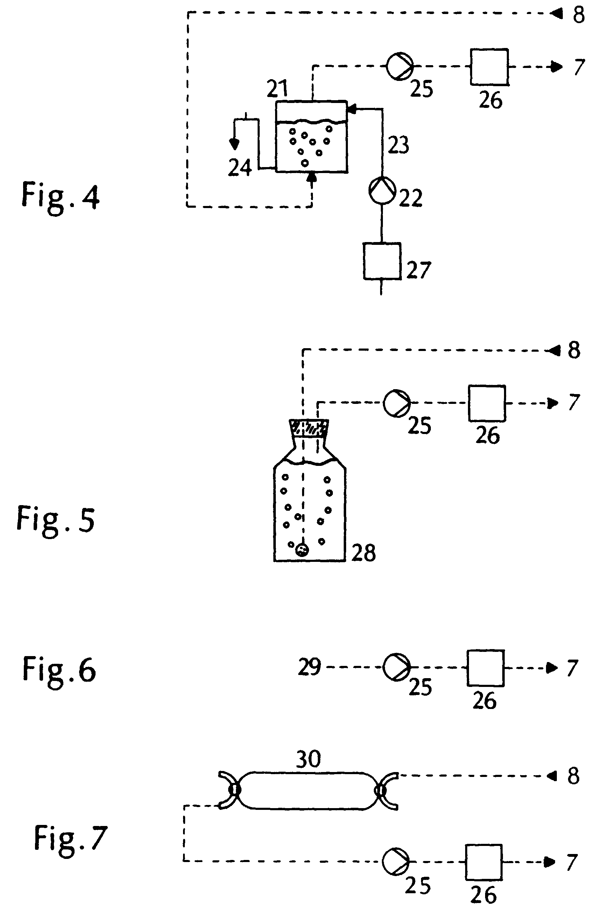

Die mit dem erfindungsgemäßen Verfahren zu untersuchenden Gasströme können beispielsweise aus folgenden in den Figuren 4 bis 7 dargestellten Quellen stammen.

In Figur 4 wird in einen Phasentauscher (21) mit einer Pumpe (22) über eine Leitung (23) eine flüssige Phase zugeführt. Über den Schwanenhalsablauf (24) verläßt die flüssige Phase den Phasentauscher. Der über die Gas-Ableitung (8) den UV-Reaktor oder das Vorratsgefäß verlassende Gasstrom (siehe Figuren 1 bis 3) wird unten in den Phasentauscher geführt und durchperlt die darin befindliche Flüssigkeit. Im oberen Teil des Phasentauschers wird der Gasstrom mit einer Pumpe (25) abgesaugt und über die Gas-Zuleitung (7) in den UV-Reaktor oder das Vorratsgefäß geleitet. Enthält der Gasstrom hinter dem Phasentauscher Kohlendioxid, kann es vorteilhaft sein, diesen Inhaltsstoff über einen CO₂-Adsorber (26) zu entfernen. Auf diesen CO₂-Adsorber kann verzichtet werden, wenn der pH-Wert der flüssigen Phase vor der Zugabe in den Phasentauscher im Behälter (27) so eingestellt wird, daß der Gasstrom, der durch den Phasentauscher geführt wird, keine ohne UV-Bestrahlung ionenbildenden Verbindungen aus der flüssigen Phase austreiben kann.

In Figur 5 befindet sich in einem geschlossenen Gefäß (28) eine flüssige Phase. Der über die Gas-Ableitung (8) zurückgeführte Gasstrom wird am Boden des Gefäßes (28) in die flüssige Phase eingeperlt, im oberen Teil des Gefäßes mit der Pumpe (25) abgesaugt und über die Gas-Zuleitung (7), ggf. über einen CO₂-Adsorber (26), in den UV-Reaktor oder das Vorratsgefäß gefördert.

In Figur 6 wird mit einer Pumpe (25) über die Leitung (29) ein Gasstrom angesaugt, ggf. über einen CO₂-Adsorber (26) geführt und über die Gas-Zuleitung (7) in den UV-Reaktor oder das Vorratsgefäß gefördert. In diesem Falle wird der Gasstrom, der die nachgeschaltete Meßvorrichtung über Leitung (8) verläßt, verworfen.

In Figur 7 befindet sich in einer Gaswurst (30) eine Gasprobe. Der über die Gas-Ableitung (8) zurückgeführte Gasstrom wird mit der Pumpe (25) durch die Gaswurst gezogen und ggf. über einen CO₂-Adsorber dem UV-Reaktor oder dem Vorratsgefäß über die Gas-Zuleitung (7) zugeführt.

Das erfindungemäße Verfahren hat folgende Vorteile:
- Gasströme können kontinuierlich in einfacher Weise und mit hoher Empfindlichkeit auf organische Inhaltsstoffe untersucht werden.
- Durch das Verfahren werden alle - kohlenstoffhaltige und nichtkohlenstoffhaltige - Zersetzungsprodukte der organischen Verbindungen nachgewiesen.
- Der totale organische Kohlenstoffgehalt (TOC) eines Gasstromes kann kontinuierlich bestimmt werden.
- Nichtflüchtige ionogene Zersetzungsprodukte (z. B. Chlorid-Ionen aus Chlorkohlenwasserstoffen) können im Wasser des UV-Reaktors angereichert werden. Dadurch wird beispielsweise eine Nachweisgrenze für Chlorkohlenwasserstoffe, die in einer Gasphase vorliegen, erreicht, die im ppt-Bereich liegt.
- Das für die Analyse als Lösemittel, Transportmittel und Reaktionspartner benötigte Wasser mit extrem hohem Reinheitsgrad ist durch den Kreislaufbetrieb ständig verfügbar.
- Die benutzten Vorrichtungen sind kalibrierfähig, wenn man eine Lösung verwendet, für die Konzentration und Art der enthaltenen zersetzungsfähigen Kohlenstoffverbindungen bekannt sind, und durch diese Lösung entsprechend den Figuren 4 und 5 einen Trägergasstrom schickt, der anschließend in einer Vorrichtung entsprechend den Figuren 1 bis 3 untersucht wird.

Das erfindungsgemäße Verfahren wird durch folgende Beispiele erläutert.

### Beispiel 1

### Untersuchung einer wäßrigen Phase mit bekanntem Trichlormethan-Gehalt

Bei dieser Untersuchung wird die in Figur 1 beschriebene Vorrichtung verwendet. Auf den CO₂-Nachweis im Trägergasstrom (CO₂-freie Luft), der den UV-Reaktor verläßt, wird verzichtet. Der dem UV-Reaktor zugeführte Gasstrom wird mit der in Figur 4 beschriebenen Vorrichtung hergestellt. Aus einem (in Figur 4 nicht dargestellten) Vorratsgefäß wird die zu untersuchende wäßrige Phase (Trinkwasser, CKW-frei) entnommen und mit einem Durchsatz von 10 Liter/Stunde durch den Phasentauscher (Inhalt: 300 ml) gepumpt. Der durch den Phasentauscher geführte Gasstrom (100 Liter/Stunde) wird vor dem Einleiten in den UV-Reaktor über einen CO₂-Adsorber (Inhalt: 500 ml) geleitet. Als CO₂-Adsorptionsmittel werden Natronkalk-Plätzchen mit Indikator (Artikel 6839, Merck Darmstadt) verwendet. Die Natronkalk-Plätzchen werden vor dem Einfüllen in das CO₂-Adsorptionsgefäß zunächst in einem Luftstrom von enthaltenem staubförmigen Natronkalk befreit und anschließend mit Wasser befeuchtet.

Der UV-Reaktor (Wasserinhalt: 50 ml) ist mit einem Quecksilber-Niederdruck-Dampfstrahler mit einer Leistung von 25 Watt bestückt. Das Kreislaufwasser in der Vorrichtung nach Figur 1 wird vor dem Einleiten in den UV-Reaktor über einen Gesamtionenaustauscher (Inhalt: 30 ml Kationenaustauscher, Typ Lewatit S 100 G1 und 70 ml Anionenaustauscher, Typ Lewatit M 600 MB; Hersteller: Bayer AG) geführt. Als Ionendetektor hinter dem UV-Reaktor wird eine temperaturkompensierte Leitfähigkeitsmeßzelle verwendet.

Der Wert für die Umlaufmenge des Kreislaufwassers wird stufenweise von 0,18 Liter/Stunde auf 3,0 Liter/Stunde erhöht, und bei jeder Einstellung wird die zugehörige Leitfähigkeit des Kreislaufwassers hinter dem UV-Reaktor gemessen. Im gesamten Bereich der Umlaufmenge hat sich eine Leitfähigkeit von 0,1 µS•cm⁻¹ im Kreislaufwasser ergeben. In der aus Figur 4 (mit nicht dargestelltem Vorratsgefäß) und Figur 1 zusammengesetzten Apparatur hat sich also ein definierter Ausgangszustand eingestellt.

In dem untersuchten Wasser des (in Figur 4 nicht dargestellten) Vorratsgefäßes werden anschließend 30 ppb Trichlormethan gelöst, bevor dieses Wasser durch den Phasentauscher geführt wurde. Die Umlaufmenge des Kreislaufwassers durch den UV-Reaktor wird wiederum stufenweise verändert und die zugehörige Leitfähigkeit gemessen. Es haben sich folgende von der Umlaufmenge abhängigen Meßwerte ergeben:

| Kreislaufwasser durch den UV-Reaktor | |
|---|---|
| Umlaufmenge Liter/Stunde | Leitfähigkeit µS/cm |
| 3,0 | 0,37 |
| 1,46 | 0,54 |
| 0,84 | 0,85 |
| 0,65 | 1,00 |
| 0,18 | 2,80 |

Bei Verringerung der Umlaufmenge des Kreislaufwassers werden bei sonst unveränderten Bedingungen die bei der Zersetzung von Trichlormethan entstandenen Chlorid-Ionen im Wasser des UV-Reaktors angereichert und bewirken eine Erhöhung des Meßeffektes.

### Beispiel 2

### Untersuchung einer wäßrigen Phase mit bekannten unterschiedlichen Trichlormethan-Gehalten

Die Untersuchung nach Beispiel 1 wird wiederholt, jedoch wird die Umlaufmenge des Kreislaufwassers durch den UV-Reaktor auf einen konstanten Wert von 0,2 Liter/Stunde eingestellt. In dem zu untersuchenden Wasser des Vorratsgefäßes werden jeweils 3 ppb, 6 ppb und 9 ppb Trichlormethan gelöst, bevor dieses Wasser durch den Phasentauscher geführt wurde. Die jeweils zugehörige Leitfähigkeit des Kreislaufwassers hinter dem UV-Reaktor wurde gemessen. Es ergaben sich folgende, von der Trichlormethan-Konzentration abhängige Meßwerte:

| Konzentration Trichlormethan ppb | Leitfähigkeit µS/cm |
|---|---|
| 0 | 0,20 |
| 3 | 0,44 |
| 6 | 0,67 |
| 9 | 0,91 |

Die Leitfähigkeit des Kreislaufwassers hinter dem UV-Reaktor erhöht sich proportional zur Trichlormethan-Konzentration im zu untersuchenden Wasser.

## Patentansprüche

1. Verfahren zum Nachweisen von zersetzungsfähigen organischen Kohlenstoffverbindungen, die in einer Gasphase vorliegen, gekennzeichnet durch
- kontinuierliches Durchleiten des vorgelegten zu untersuchenden gasförmigen Stromes durch eine begrenzte Menge Wasser, die sich in einem Wasserkreislauf (6) befindet, der mindestens einen UV-Reaktor (1), einen Ionendetektor (2), einen Ionenaustauscher (4) und eine Pumpe (5) enthält,
- kontinuierliches Bestrahlen der kontinuierlich umgepumpten begrenzten Wassermenge durch den Wasserkreislauf (6) im UV-Reaktor (1) mit UV-Licht, wobei die über die Gasphase in den Wasserkreislauf eingeleiteten zersetzungsfähigen organischen Kohlenstoffverbindungen zersetzt werden,
- kontinuierliches Nachweisen der im Kreislaufwasser enthaltenen Zersetzungsprodukte mit dem in dieses Wasser eintauchenden Ionendetektor (2), bevor das Kreislaufwasser den Ionenaustauscher (4) erreicht,
- Entfernen der Ionen aus dem Kreislaufwasser mittels eines Ionenaustauschers (4), wobei
- während des Nachweisens der Zersetzungsprodukte das Wasser im Kreislauf geführt wird.

2. Verfahren nach Anspruch 1, gekennzeichnet durch
- kontinuierliches Durchleiten des vorgelegten zu untersuchenden gasförmigen Stromes durch den im Wasserkreislauf (6) vorhandenen und von Wasser kontinuierlich durchströmten UV-Reaktor (1).

3. Verfahren nach Anspruch 1, gekennzeichnet durch
- kontinuierliches Durchleiten des vorgelegten zu untersuchenden gasförmigen Stromes durch ein im Wasserkreislauf (6) vorhandenes und von Wasser kontinuierlich durchströmtes Ausgleichsgefäß (3).

4. Verfahren nach Anspruch 1, gekennzeichnet durch
- kontinuierliches Durchleiten des vorgelegten zu untersuchenden gasförmigen Stromes durch ein im Wasserkreislauf (6) vorhandenes und von Wasser kontinuierlich durchströmtes Ausgleichsgefäß (3), wobei
- der das Ausgleichsgefäß (3) verlassende gasförmige Strom anschliessend durch den im Wasserkreislauf (6) vorhandenen und von Wasser kontinuierlich durchströmten UV-Reaktor (1) geleitet wird.

5. Verfahren nach den Ansprüchen 1 bis 4, gekennzeichnet durch
- Nachweisen der bei der Zersetzung im UV-Reaktor (1) gebildeten Zersetzungsprodukte
- in der gasförmigen Phase, die den UV-Reaktor (1) verläßt, mittels eines Kohlendioxid-Detektors (9), oder
- sowohl in der begrenzten Wassermenge mittels eines Ionendetektors (2) als auch in der gasförmigen Phase, die den UV-Reaktor (1) verläßt, mittels eines Kohlendioxid-Detektors (9).

6. Verfahren nach den Ansprüchen 1 bis 5, wobei
- der vorgelegte zu untersuchende gasförmige Strom, der die nachzuweisenden zersetzungsfähigen organischen Kohlenstoffverbindungen enthält, hergestellt wird mittels eines von Kohlenstoffverbindungen freien Trägergases, mit dem die zunächst in einer flüssigen Phase vorliegenden nachzuweisenden zersetzungsfähigen organischen flüchtigen Kohlenstoffverbindungen aus der flüssigen Phase ausgetrieben werden.

7. Verfahren nach den Ansprüchen 1 bis 6, wobei
- das zu untersuchende gasförmige Probegut hergestellt wird durch Durchleiten eines Trägergasstromes, der frei von kohlenstoffhaltigen Verbindungen ist, durch eine flüssige Phase, die die zersetzungsfähigen Kohlenstoffverbindungen enthält, wobei die flüchtigen organischen Verbindungen in den Trägergasstrom übergehen und wobei in dieser flüssigen Phase der pH-Wert auf einen Wert eingestellt ist, bei dem der Trägergasstrom nur solche Verbindungen aus der flüssigen Phase ausstrippen kann, die ohne UV-Bestrahlung in wäßriger Lösung keine ionogenen Verbindungen bilden.

8. Verfahren nach den Ansprüchen 1 bis 7, wobei
- ein von molekularem Sauerstoff freies Trägergas, bevorzugt Stickstoff, oder ein Edelgas wie Helium oder Argon, verwendet werden.

## Claims

1. A method for detecting decomposable organic carbon compounds which are present in a gas phase, characterized by
- continuously passing the gaseous stream introduced to be investigated through a restricted amount of water which is situated in a water circuit (6) which comprises at least one UV reactor (1), an ion detector (2), an ion exchanger (4) and a pump (5),
- continuously irradiating the restricted amount of water which is continuously circulated by pumping through the water circuit (6) in the UV reactor (1) with UV light, the decomposable organic carbon compounds introduced into the water circuit via the gas phase being decomposed,
- continuously detecting the decomposition products present in the circulated water using the ion detector (2) immersed in this water before the circulated water reaches the ion exchanger (4),
- removing the ions from the circulated water by means of an ion exchanger (4),
- the water being circulated during detection of the decomposition products.

2. A method according to claim 1, characterized by
- continuously passing the gaseous stream introduced to be investigated through the UV reactor (1) which is present in the water circuit (6) and through which water continuously flows.

3. A method according to claim 1, characterized by
- continuously passing the gaseous stream introduced to be investigated through an equilibrating vessel (3) which is present in the water circuit (6) and through which water continuously flows.

4. A method according to claim 1, characterized by
- continuously passing the gaseous stream introduced to be investigated through an equilibrating vessel (3) which is present in the water circuit (6) and through which water continuously flows,
- the gaseous stream leaving the equilibrating vessel (3) then being passed through the UV reactor (1) which is present in the water circuit (6) and through which water continuously flows.

5. A method according to any of claims 1 to 4, characterized by
- detecting the decomposition products formed on decomposition in the UV reactor (1)
- in the gaseous phase which leaves the UV reactor (1) by means of a carbon dioxide detector (9), or
- both in the restricted amount of water by means of an ion detector (2) and in the gaseous phase which leaves the UV reactor (1) by means of a carbon dioxide detector (9).

6. A method according to any of claims 1 to 5, where
- the gaseous stream introduced to be investigated, which contains the decomposable organic carbon compounds to be detected, is produced using a carbon-compound-free carrier gas, by means of which decomposable organic volatile carbon compounds to be detected which are initially present in a liquid phase are expelled from the liquid phase.

7. A method according to any of claims 1 to 6, where
- the gaseous sample material to be investigated is produced by passing a carrier gas stream which is free of carbon-containing compounds through a liquid phase which contains the decomposable carbon compounds, the volatile organic compounds transferring to the carrier gas stream and the pH in this liquid phase being adjusted to a value at which the carrier gas stream can strip only those compounds from the liquid phase which do not form ionic compounds in aqueous solution in the absence of UV irradiation.

8. A method according to any of claims 1 to 7, where
- a molecular oxygen-free carrier gas, preferably nitrogen, or a noble gas such as helium or argon, is employed.

## Revendications

1. Procédé de détection de composés carbonés organiques décomposables qui sont présents dans une phase gazeuse,
caractérisé par
- une traversée en continu du courant gazeux à analyser introduit par une quantité d'eau limitée qui se trouve dans un circuit d'eau (6) qui renferme au moins un réacteur à UV (1), un détecteur d'ions (2), un échangeur d'ions (4) et une pompe (5),
- une irradiation en continu d'une certaine quantité d'eau, transvasée par pompage en continu à travers le circuit d'eau (6) dans le réacteur à UV (1), avec de la lumière UV, dans lequel les composés carbonés organiques décomposables introduits dans le circuit d'eau au-dessus de la phase gazeuse sont décomposés,
- une détection en continu des produits de décomposition contenus dans l'eau du circuit à l'aide du détecteur d'ions (2) immergé dans cette eau, avant que l'eau du circuit atteigne l'échangeur d'ions (4),
- l'élimination des ions de l'eau du circuit à l'aide d'un échangeur d'ions (4) dans lequel pendant la détection des produits de décomposition, l'eau est amenée dans le circuit.

2. Procédé selon la revendication 1,
caractérisé par :
- la traversée en continu du courant gazeux à analyser, introduit, par le réacteur à UV (1) présent dans le circuit d'eau (6) et traversé en continu par l'eau.

3. Procédé selon la revendication 1,
caractérisé par :
- la traversée en continu du courant gazeux à analyser introduit, par un récipient d'équilibrage (3) présent dans le circuit d'eau (6) et parcouru en continu par de l'eau.

4. Procédé selon la revendication 1,
caractérisé par :
- la traversée en continu du courant gazeux à analyser introduit par un récipient d'équilibrage (3) traversé en continu par de l'eau, et présent dans le circuit d'eau (6)
- le courant gazeux qui quitte le récipient d'équilibrage (3) est conduit ensuite à travers le réacteur à UV (1) traversé en continu par de l'eau et présent dans le circuit d'eau (6).

5. Procédé selon les revendications 1 à 4,
caractérisé par :
- la détection des produits de décomposition formés au cours de la décomposition dans le réacteur à UV (1),
- dans la phase gazeuse qui quitte le réacteur à UV (1) à l'aide d'un détecteur à gaz carbonique (9) ou
- aussi bien dans la certaine quantité d'eau à l'aide d'un détecteur d'ions (2) que dans la phase gazeuse qui quitte le réacteur à UV(1), à l'aide d'un détecteur à gaz carbonique (9).

6. Procédé selon les revendications 1 à 5,
caractérisé en ce que
- le courant gazeux à analyser, introduit, qui renferme les composés carbonés organiques décomposés détectés, est produit à l'aide d'un gaz porteur dépourvu de composés carbonés avec lequel les composés carbonés volatils organiques décomposables à détecter, présents en premier lieu dans une phase liquide, sont chassés de la phase liquide.

7. Procédé selon les revendications 1 à 6,
caractérisé en ce que
- le produit d'échantillon gazeux à analyser est produit par la traversée d'un courant de gaz porteur qui est dépourvu de composés carbonés, par une phase liquide qui renferme les composés carbonés décomposables, dans laquelle les composés organiques volatils, passent dans le courant de gaz porteur et, dans cette phase liquide, la valeur du pH est ajustée à une valeur à laquelle le courant de gaz porteur peut extraire seulement les composés de la phase aqueuse ne formant, sans irradiation par les UV en solution aqueuse, aucun composé ionogène.

8. Procédé selon les revendications 1 à 7,
caractérisé en ce qu'
un gaz porteur débarrassé d'oxygène moléculaire, de préférence de l'azote ou un gaz rare comme l'hélium ou l'argon, est utilisé.
